# EUROPEAN PATENT APPLICATION

(11) **EP 4 040 308 A1**
(43) Date of publication of application: **10.08.2022**
(21) Application number: 19947464.4
(22) Date of filing: 01.10.2019
(51) Int. Cl.: G06F 16/28

(54) **LEARNING METHOD, LEARNING DEVICE, LEARNING PROGRAM, PREDICTION METHOD, PREDICTION DEVICE, AND PREDICTION PROGRAM**

(71) Applicant: FUJITSU LIMITED, Kawasaki-shi, Kanagawa 211-8588 (JP)
(72) Inventor: UKAI, Takanori, Kawasaki-shi, Kanagawa 211-8588 (JP); OKAJIMA, Seiji, Kawasaki-shi, Kanagawa 211-8588 (JP)
(74) Representative: Hoffmann Eitle
(86) International application number: PCT/JP2019/038822
(87) International publication number: WO 2021/064879

(57) **Abstract**

A training apparatus obtains RDF data including subjects, predicates, and objects, inputs a subject, a predicate, and an object of a first record of the obtained RDF data (S31), and determines a predicate of a second record that has been previously input, the predicate having the same character string as the subject or object of the first record (S36). The training apparatus generates training data including RDF data having the subject or object of the first record and the predicate of the second record that have been associated with each other (S38). The training apparatus performs training for the generated training data so that a vector resulting from addition of a vector of the predicate to a vector of the subject associated with the RDF data becomes closer to a vector of the object associated with the RDF data, and thereby enables improvement in prediction accuracy for searching through the RDF data (S14, S24).

## Description

### [Technical Field]

The present invention relates to training methods, for example.

### [Background Art]

A technology (referred to as "TransE") for embedding relative information into knowledge graphs to predict links in the knowledge graphs has been disclosed, the relative information being formed of three sets of (triple) data (see, for example, Non-Patent Literature 1). Examples of this relative information include resource description frameworks (RDFs). An RDF indicates a data structure for describing metadata on web information and has, as a group, three sets of data that are an entity, a property, and an entity. These entity, property, and entity are expressed by having three elements, a subject, a predicate, and an object, as relative information. These subject, predicate, and object are relative information having a relation that "the predicate of the subject is the object".

In TransE, training for the embedding of vectors of entities and properties is performed based on a set S of triple data (h, r, t) having two entities h and t belonging to E (a set of entities) and a property r belonging to R (a set of properties). TransE is a technology for obtaining a data structure by embedding a set of knowledge graphs into vector space and performing vector transformation using a machine learning technique, the set having groups (triples) each having three sets of data, (h, r, t). For respective vector representations Vₕ, Vᵣ, and Vₜ of the triple data (h, r, t), the data structure herein refers to a data structure in which Vₕ + Vᵣ becomes equal to Vₜ as much as possible.

Accordingly, using a data structure trained by TransE enables a calculation, like Vₕ + Vᵣ ≈Vₜ), thus enabling the prediction of t corresponding to Vₕ + Vᵣ. Furthermore, using a data structure trained by TransE enables the prediction of h corresponding to Vₜ - Vᵣ and r corresponding to Vₜ - Vₕ.

### [Citation List]

### [Patent Citation]

Patent Literature 1: Japanese Laid-open Patent Publication No. 2019-032704
Patent Literature 2: Japanese Laid-open Patent Publication No. 2016-099705
Patent Literature 3: Japanese Laid-open Patent Publication No. 2018-194944
Patent Literature 4: Japanese Laid-open Patent Publication No. 2017-076403

### Non-Patent Literature

Non-Patent Literature 1: Antonine Bordes et al., "Translating Embeddings for Modeling Multi-relational Data."

### [Summary of Invention]

### [Technical Problem]

However, a character string used in a predicate of an RDF may be used in a subject or object. Furthermore, a character string used in a subject or object may be used in a predicate.

In either of these cases, because a set E of entities indicating subjects and objects and a set R of properties indicating predicates are independent of each other in Trans E, even if the same character string is used for a subject or object and a predicate, their vectors differ from each other. Therefore, because a predicate cannot be treated as a subject or an object due to characteristics of data structures of RDFs in TransE, predicting a missing part of data on the RDF is difficult. TransE has a problem that data prediction accuracy is difficult to improve even if RDFs have trained data structures are used.

In particular, RDF data are known to have many missing parts, and the prediction accuracy of searching is thus desired to be improved to reduce search failures conveniently.

In one aspect, an object of the present invention is to improve prediction accuracy of searching through data on RDFs.

### [Solution to Problem]

According to an aspect of the embodiments, a training method includes: obtaining resource description framework (RDF) data including subjects, predicates, and objects; inputting a subject, a predicate, and an object of a first record in the RDF data obtained; determining a predicate of a second record previously input, the predicate having the same character string as the subject or the object of the first record; generating training data including RDF data having the subject or the object of the first record and the determined predicate of the second record that have been associated with each other, by the processor; and performing training for the generated training data so that a vector resulting from addition of a vector of the predicate to a vector of the subject associated with the RDF data becomes closer to a vector of the object associated with the RDF data..

### [Advantageous Effects of Invention]

According to one embodiment, the prediction accuracy of searching through data on RDFs can be improved.

### [Brief Description of Drawings]

FIG. 1 is a functional block diagram illustrating a configuration of a training system according to an embodiment.
FIG. 2 is a diagram illustrating an example of RDF data according to the embodiment.
FIG. 3 is a diagram illustrating a knowledge graph according to the embodiment.
FIG. 4A is a diagram illustrating an initializing process according to the embodiment.
FIG. 4B is a diagram illustrating the example of the initializing process according to the embodiment.
FIG. 5A is a diagram illustrating an example of a training process according to the embodiment.
FIG. 5B is a diagram illustrating the example of the training process according to the embodiment.
FIG. 5C is a diagram illustrating the example of the training process according to the embodiment.
FIG. 5D is a diagram illustrating the example of the training process according to the embodiment.
FIG. 5E is a diagram illustrating the example of the training process according to the embodiment.
FIG. 5F is a diagram illustrating the example of the training process according to the embodiment.
FIG. 5G is a diagram illustrating the example of the training process according to the embodiment.
FIG. 5H is a diagram illustrating the example of the training process according to the embodiment.
FIG. 6 is a diagram illustrating an example of a predicting process according to the embodiment.
FIG. 7A is a diagram illustrating an example of a flowchart for the training process according to the embodiment.
FIG. 7B is a diagram illustrating another example of the flowchart for the training process according to the embodiment.
FIG. 8 is a diagram illustrating an example of a flowchart for the initializing process according to the embodiment.
FIG. 9 is a diagram illustrating an example of a flowchart for the predicting process according to the embodiment.
According to the embodiment, FIG. 10 is a diagram illustrating another example of the RDF data.
FIG. 11 is a diagram illustrating a predicting process using the other example of the RDF data.
FIG. 12 is a diagram illustrating an example of an output screen according to an embodiment.
According to the embodiment, FIG. 13 is a diagram illustrating how training is performed using a neural network in the training process.
FIG. 14 is a diagram illustrating an example of a computer that executes a training program.
FIG. 15 is a reference diagram illustrating an example of training in TransE.
FIG. 16 is a reference diagram illustrating the example of the training in TransE.
FIG. 17 is a reference diagram illustrating the example of the training in TransE.
FIG. 18 is a reference diagram illustrating an example in which prediction fails when TransE is used.
FIG. 19 is a reference diagram illustrating the example in which prediction fails when TransE is used.
FIG. 20 is a reference diagram illustrating the example in which prediction fails when TransE is used.

### [Embodiments for Carrying Out the Invention]

Embodiments of a training method, a training apparatus, a training program, a predicting method, a predicting apparatus, and a predicting program disclosed by this application will hereinafter be described in detail based on the drawings. These embodiments do not limit the present invention.

First of all, "TransE" for embedding relative information formed of plural sets of data into a knowledge graph to predict a link in the knowledge graph will be described. "TransE" is a technology for obtaining a predetermined data structure by embedding a set of knowledge graphs having three sets of data as a group into vector space and performing vector transformation using a machine learning technique. The predetermined data structure refers to a data structure in which, in a case where a group of sets of data are (h, r, t) (h: subject; r: predicate; and t: object), Vₕ + Vᵣ becomes equal to Vₜ as much as possible, for respective vectors V of h, r, and t. Accordingly, using a data structure trained by TransE enables a calculation, like Vₕ + Vᵣ ≈ Vₜ), thus enabling the prediction of t corresponding to Vₕ + Vᵣ. Furthermore, using a data structure trained by TransE enables the prediction of h corresponding to Vₜ - Vᵣ and r corresponding to Vₜ - Vₕ.

Data having a data format describing a relation that "the predicate of the subject is the object", will be referred to as resource description framework (RDF) data, the data has, as a group, three sets of data, (h, r, t). Furthermore, RDF data has been described as a data structure having, as a group, three sets of data that are a subject, a predicate, and an object, but a subject and an object may be referred to as "entities" and a predicate may be referred to as a "property", as appropriate.

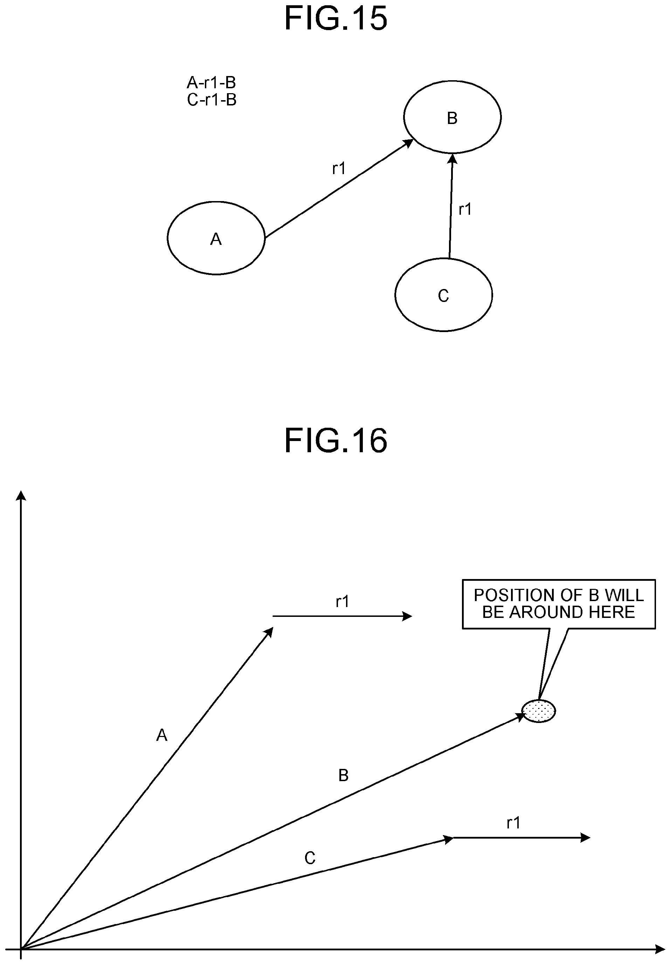

An example of training in TransE will now be described by reference to FIG. 15 to FIG. 17. FIG. 15 to FIG. 17 are reference diagrams illustrating the example of the training in TransE. The example is a case where RDF data are (A, r1, B) and (C, r1, B).

A graph illustrated in FIG. 15 represents the connection between data sets in the RDF data used for the training in TransE, using a knowledge graph. That is, in TransE, mapping is performed so that "A" + "r1" becomes closer to "B", and "C" + "r1" to "B". Training for this mapping will hereinafter be described with respect to two-dimensions.

As illustrated in FIG. 16, a vector V_{A} of "A", a vector of Vᵣ₁ of "r1", a vector V_{B} of "B", and a vector V_{C} of "C" are initialized with random numbers and located in two-dimensional space.

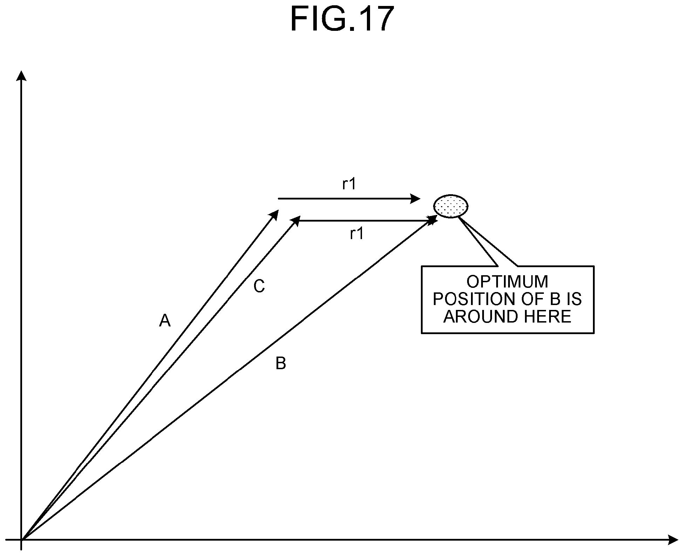

Next, as illustrated in FIG. 17, the respective vectors are optimized by training so that V_{A} + Vᵣ₁ becomes closer to V_{B} and V_{C} + Vᵣ₁ to V_{B}. As a result, the position of B is optimized by the training. That is, the training is performed so that the distance between the position indicated by V_{A} + Vᵣ₁ and the position indicated by V_{B} will be within a predetermined range (a score), and the distance between the position indicated by V_{C} + Vᵣ₁ and the position indicated by V_{B} will be within the predetermined range (a score).

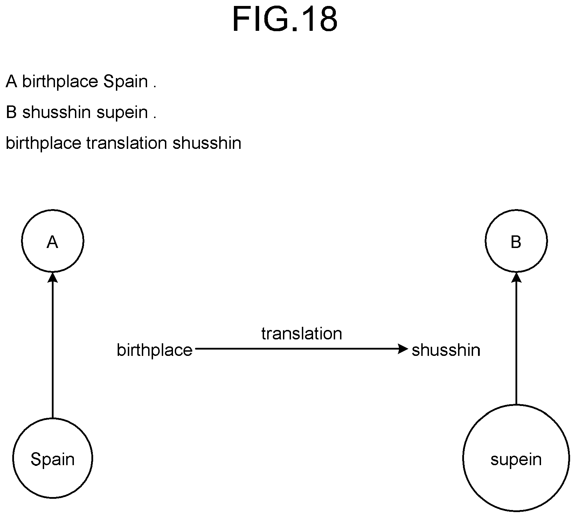

Next, an example in which prediction fails in a case where trained RDF data that TransE has trained is used will be described by reference to FIG. 18 to FIG. 20. FIG. 18 to FIG. 20 are reference diagrams illustrating an example in which prediction fails when TransE is used. In this example, there are (A, birthplace, Spain), (B, shusshin, supein), and (birthplace, translation, shusshin) in the RDF data.

FIG. 18 illustrates a knowledge graph representing a connection between sets of data in the RDF data trained by TransE.

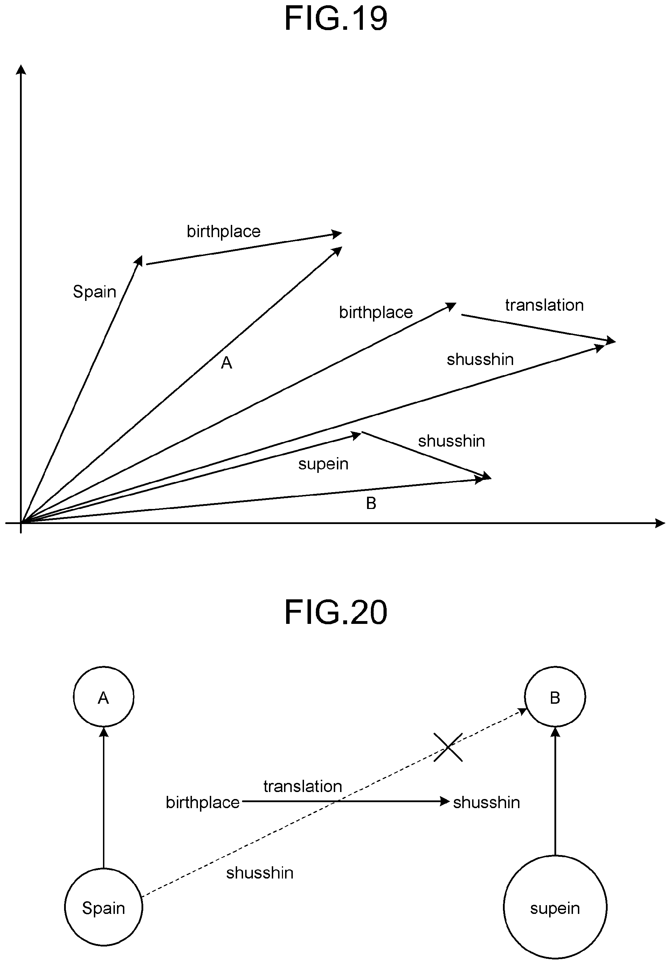

As illustrated in FIG. 19, respective vectors of (A, birthplace, Spain), (B, shusshin, supein), and (birthplace, translation, shusshin) trained by TransE are represented in two-dimensional space. In TransE, a set of entities indicating subjects and objects and a set of properties indicating predicates are independent of each other. Therefore, "birthplace" indicated by a property and "birthplace" indicated by an entity are represented by entirely different vectors even though their character strings are the same. "Shusshin" indicated by a property and "shusshin" indicated by an entity are represented by entirely different vectors even though these character strings are the same.

In TransE, the fact that "A" and "Spain" have a relation is learned but the fact that "A" and "supein" have a relation is not learned. Furthermore, the fact that "B" and "supein" have a relation is learned but the fact that "B" and "Spain" have a relation is not learned.

That is, as illustrated in FIG. 20, for example, the fact that "shusshin" of "B" is "Spain" is unable to be predicted. Furthermore, the fact that "shusshin" of "A" is "supein" is unable to be predicted, although this is not illustrated in the drawings. That is, "A" and "B" have the same "shusshin", "Spain" and "supein" have a "translation" relation, and "B" and "Spain" are thus desired to have a close relation, but "B" and "Spain" end up having a distant relation and the fact that "shusshin" of "B" is "Spain" is unable to be predicted. Similarly, "A" and "B" have the same "shusshin", "Spain" and "supein" have a "translation" relation, and "A" and "supein" are thus desired to have a close relation, but "A" and "supein" end up having a distant relation and the fact that "shusshin" of "A" is "supein" is unable to be predicted. That is, TransE has a problem that data prediction accuracy is difficult to be improved even if RDF data having a trained data structure is used.

Therefore, training systems that improve data prediction accuracy using RDF data with trained data structures will be described in the following embodiments. Embodiments

### Configuration of Training System

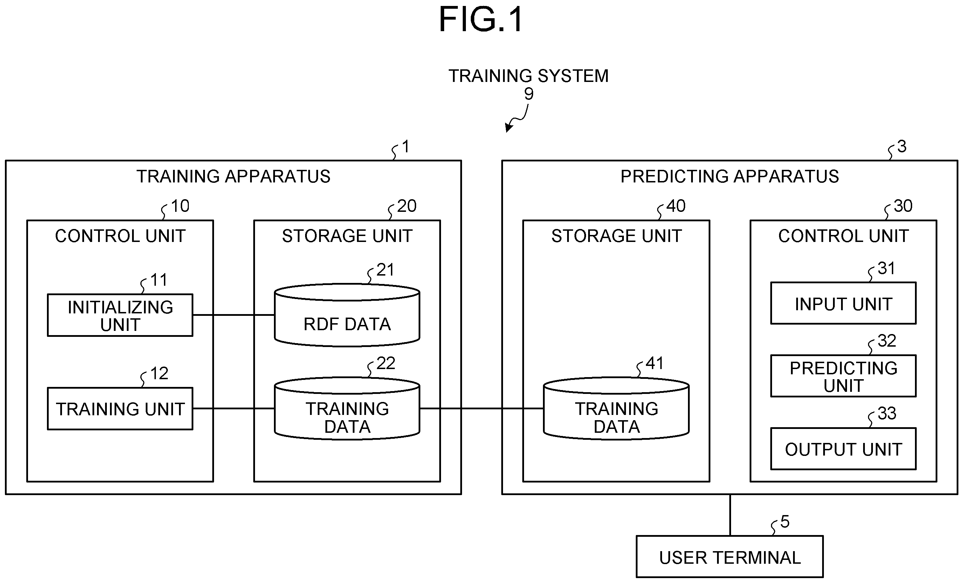

FIG. 1 is a functional block diagram illustrating a configuration of a training system according to an embodiment. A training system 9 includes a training apparatus 1 and a predicting apparatus 3. The training apparatus 1 performs training so that a vector resulting from the addition of a vector of a predicate to a vector of a subject becomes closer to a vector of an object, in RDF data formed of subjects, predicates, and objects. The training apparatus 1 performs this training such that even in a case where the character string of a predicate is used as a subject or object, these character strings are used as the same vector. Furthermore, when input data is input to the predicting apparatus 3, the predicting apparatus 3 predicts a prediction target based on a result of the training of the RDF data, the input data being for a subject, a predicate, and an object and having any of a subject, a predicate, or an object as the prediction target. RDF data has been described as a data structure formed of three sets of data: a subject, a predicate, and an object, but a subject or an object may hereinafter be referred to as an "entity" and a predicate as a "property".

The training apparatus 1 has a control unit 10 and a storage unit 20.

The control unit 10 corresponds to an electronic circuit, such as a central processing unit (CPU). The control unit 10 has an internal memory for storing therein programs prescribing various processing procedures, and control data; and the control unit 10 executes various types of processing by using them. The control unit 10 has an initializing unit 11 and a training unit 12. The initializing unit 11 is an example of an obtaining unit, a determining unit, and a generating unit.

The storage unit 20 is, for example: a semiconductor memory element, such as a RAM or a flash memory; or a storage device, such as a hard disk or an optical disk. The storage unit 20 has therein RDF data 21 and training data 22.

The RDF data 21 indicates a data structure for describing metadata on the web information and has, as one group, three sets of data that are a subject, a predicate, and an object. That is, the RDF data 21 has, as a group, three sets of data that are an entity, a property, and an entity. Each group of the RDF data 21 has a relation that "the predicate of the subject is the object". For the RDF data 21, this relation can be represented by a directed and labelled knowledge graph. The three sets of data that are the subject, predicate, and object groups are called a "triple".

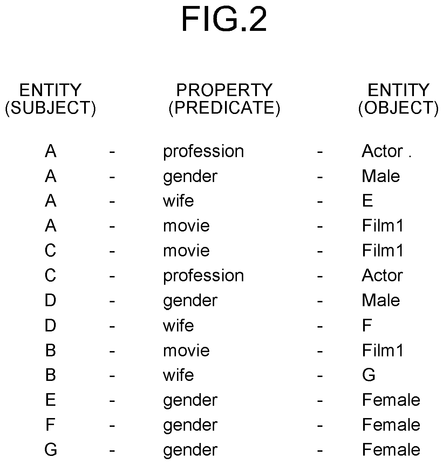

An example of the RDF data 21 will now be described by reference to FIG. 2. FIG. 2 is a diagram illustrating an example of RDF data according to the embodiment. FIG. 2 illustrates the RDF data 21 having, as one group, three sets of data that are an entity (a subject), a property (a predicate), and an entity (an object). Each group has the relation that "the predicate of the subject is the object".

For example, (A, profession, Actor) has been stored as (subject, predicate, object) in the RDF data 21. This (A, profession, Actor) has a relation that "'profession' of 'A' is 'Actor'". Furthermore, (A, gender, Male) has been stored as (subject, predicate, object). This (A, gender, Male) has a relation that "'gender' of 'A' is 'Male'".

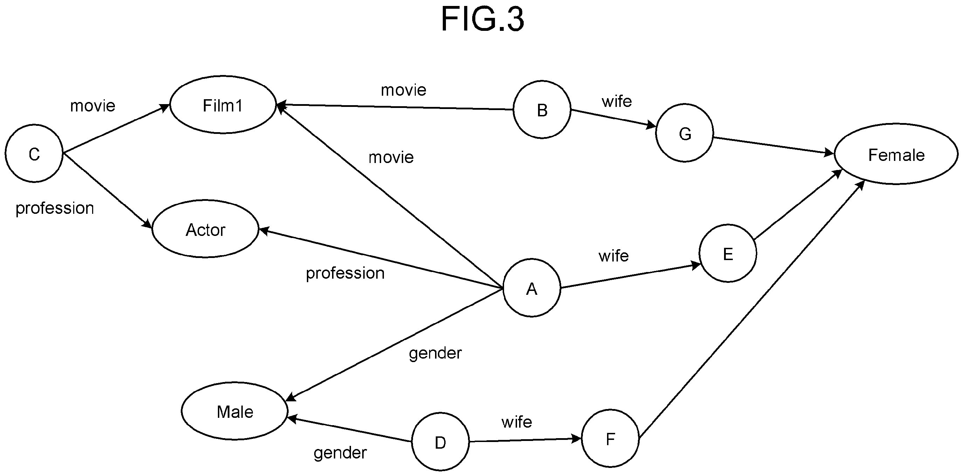

FIG. 3 represents the RDF data 21 illustrated in FIG. 2 as a knowledge graph. FIG. 3 is a diagram illustrating a knowledge graph according to the embodiment. A subject and an object of (subject, predicate, object) are represented by nodes and the subject is represented as a starting point and the object as an end point. A predicate of (subject, predicate, object) is indicated by a label below an arrow.

For example, in a case where (subject, predicate, object) is (A, profession, Actor), the node, "A", is a starting point, the node, "Actor", is an end point, and "profession" is a label. In a case where (subject, predicate, object) is (A, gender, Male), the node, "A", is a starting point, the node, "Male", is an end point, and "gender" is a label.

In FIG. 1, to which reference is made again, the training data 22 is data resulting from training of the RDF data 21. For example, the training data 22 includes a set of trained vectors for respective character strings included in subjects, predicates, and objects in the RDF data 21.

The initializing unit 11 performs the initialization of vectors based on three sets of data of each group included in the RDF data 21. For example, the initializing unit 11 reads the groups included in the RDF data 21 in sequence. The initializing unit 11 initializes respective vectors of three sets of data of a group that has been read with random numbers. In this initialization, in a case where the character string of an entity indicating a subject or object and the character string of a property indicating a predicate are the same, the initializing unit 11 associates the character string of the entity and the character string of the property with each other and make these character strings indicate the same vector. The initializing unit 11 repeats this initializing process until initializing processes for all of the groups included in the RDF data 21 are finished. The dimensionality of the vectors of entities included in a group and the dimensionality of the vector of the property are made the same. Furthermore, the associations generated by the initializing unit 11 are training data examples.

For all of the groups included in the RDF data 21, the training unit 12 performs training so that a vector resulting from addition of the vector of a property indicating a predicate to the vector of an entity indicating a subject becomes closer to the vector of an entity indicating an object. For example, in a case where the character string of the property indicating the predicate of a group has been associated with the character string of the entity indicating the subject of another group, the property and the entity of these associated character strings are calculated as the same vector. For example, the training unit 12 performs training so that a vector resulting from addition of a common vector to the vector of the entity of the subject of a certain group becomes closer to the vector of the entity indicating the object of that certain group, the common vector being a vector of an entity of another group, the entity of this other group having a character string that is the same as that of the property of that certain group. That is, the training unit 12 performs training for embedding the vectors of the entity and property based on a set of triple data (h, r, t) in which a set E1 of entities belongs to E (the whole set) and a set E2 of properties belongs to E1 (the set of entities). The training unit 12 then saves a result of the training in the training data 22. The training saved in the training data 22 includes a set of trained vectors for respective character strings included in subjects, predicates, and objects included in the RDF data 21.

The predicting apparatus 3 is connected to a user terminal 5 and has a control unit 30 and a storage unit 40.

The control unit 30 corresponds to an electronic circuit, such as a central processing unit (CPU). The control unit 30 has an internal memory for storing therein programs prescribing various processing procedures and control data; and the control unit 30 executes various types of processing by using them. The control unit 30 has an input unit 31, a predicting unit 32, and an output unit 33.

The storage unit 40 is, for example: a semiconductor memory element, such as a RAM or a flash memory; or a storage device, such as a hard disk or an optical disk. The storage unit 40 has therein training data 41. This training data 41 is the same as the training data 22 in the training apparatus 1 and description of the training data 41 will thus be omitted.

The input unit 31 inputs, from the user terminal 5, a group in the RDF data 21, the group having any of a subject, a predicate, or an object as a prediction target.

The predicting unit 32 predicts a prediction target for a group that has been input using trained vectors. For example, by using a set of trained vectors for respective character strings of the training data 22, the predicting unit 32 predicts the prediction target for the input group as follows. The predicting unit 32 obtains, from the set of trained vectors, vectors corresponding to the two character strings of the input group, the two-character strings being other than the prediction target. The predicting unit 32 then selects vectors one by one from the set of trained vectors. By using the vector selected and the vectors of the character strings other than the prediction target, the predicting unit 32 then retrieves a vector smaller than a predetermined score, the retrieved vector resulting from subtraction of the vector of an object from a vector resulting from addition of the vector of the predicate to the vector of the subject. The predicting unit 32 predicts, as the prediction target, the character string corresponding to the vector that can be retrieved. For example, in a case where what "shusshin" of "A" is to be predicted, (h, r, t) is ("A", "shusshin", t) and the object is the prediction target.

The predicting unit 32 thus retrieves a selected vector Vₜ such that a vector resulting from subtraction of the selected vector Vₜ from a vector resulting from addition of a vector Vᵣ of "shusshin" to a vector "Vₕ" of "A" becomes smaller than the score. The predicting unit 32 then predicts, as the prediction target, a character string t corresponding to the selected vector Vₜ that can be retrieved.

The output unit 33 outputs the prediction target predicted by the predicting unit 32, to the user terminal 5.

### Example of Initializing Process

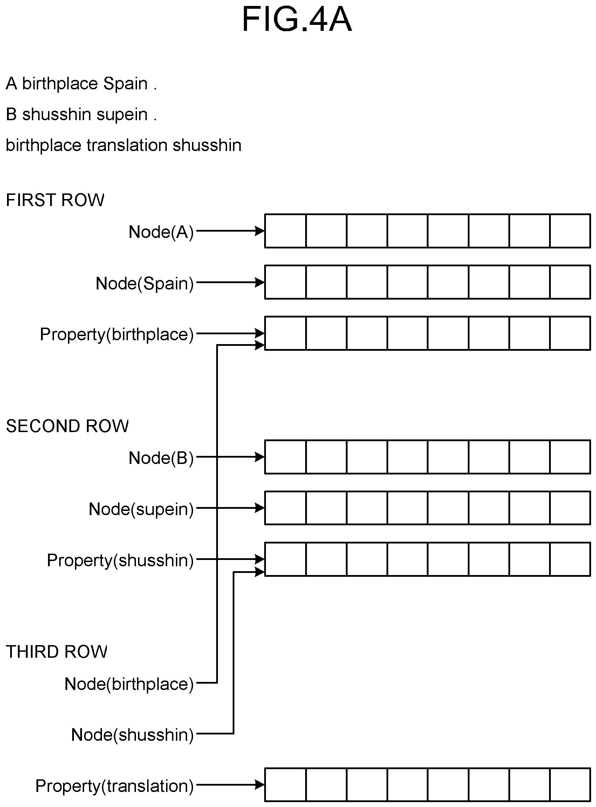

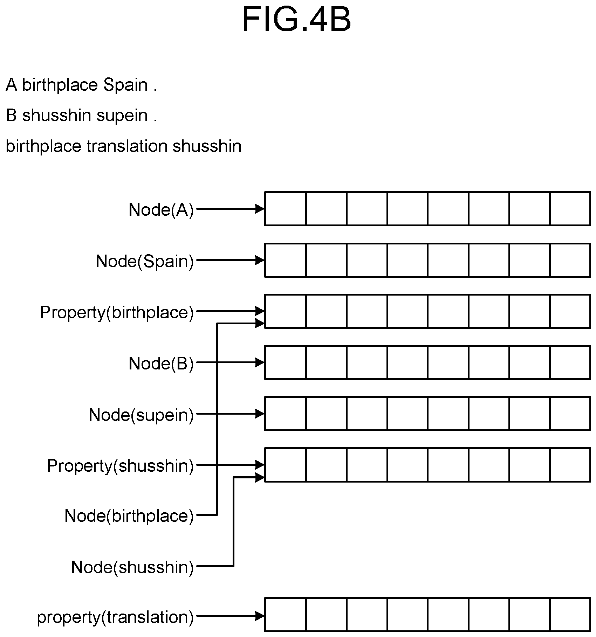

An example of the initializing process according to the embodiment will now be described by reference to FIG. 4A and FIG. 4B. FIG. 4A and 4B are diagrams illustrating the example of the initializing process according to the embodiment. A case where (subject, predicate, object) included in the RDF data 21 is (A, birthplace, Spain), (B, shusshin, supein), and (birthplace, translation, shusshin) will be described by reference to FIG. 4A and FIG. 4B. It is assumed that none of the vectors of the items in the RDF data 21 have been initialized yet. The vectors of the subjects, predicates, and objects are all assumed to be n-dimensional.

As illustrated in FIG. 4A, the initializing unit 11 reads three items of a group in a first row from the RDF data 21. Because the vector of the item, "A" (Node (A)), which is the first item, has not been initialized yet, the initializing unit 11 initializes the n-dimensional vector with a random number. Because the vector of the item, "Spain" (Node (B)), which is the third item, has not been initialized yet, the initializing unit 11 initializes the n-dimensional vector with a random number. Because the vector of the item, "birthplace" (Property (birthplace)), which is the second item, has not been initialized yet, the initializing unit 11 initializes the n-dimensional vector with a random number.

Next, the initializing unit 11 reads three items of a group in a second row from the RDF data 21, and initializes the respective vectors of "B" (Node (B)), "supein" (Node (supein)), and "shusshin" (Property (shusshin)), similarly to the first row.

Next, the initializing unit 11 reads three items of a group in a third row from the RDF data 21. Because the vector of the item, "birthplace" (Node (birthplace)), of the first item has been initialized already, the initializing unit 11 makes the node (birthplace) indicate the vector of the property (birthplace). That is, the initializing unit 11 associates the character string of the node in the third row and the character string of the property in the first row with each other and makes these character strings indicate the same vector. Furthermore, because the vector of the item, "shusshin" (Node (shusshin)), of the third item has been initialized already, the initializing unit 11 makes the node (shusshin) indicate the vector of the property (shusshin). That is, the initializing unit 11 associates the character string of the node in the third row and the character string of the property in the second row with each other and makes these character strings indicate the same vector. In addition, because the vector of the item, "translation" (Property (translation)), which is the second item, has not been initialized yet, the initializing unit 11 initializes the n-dimensional vector with a random number.

As illustrated in FIG. 4B, seven vectors are generated in this example. The associations generated as described above are an example of training data.

### Example of Training Process

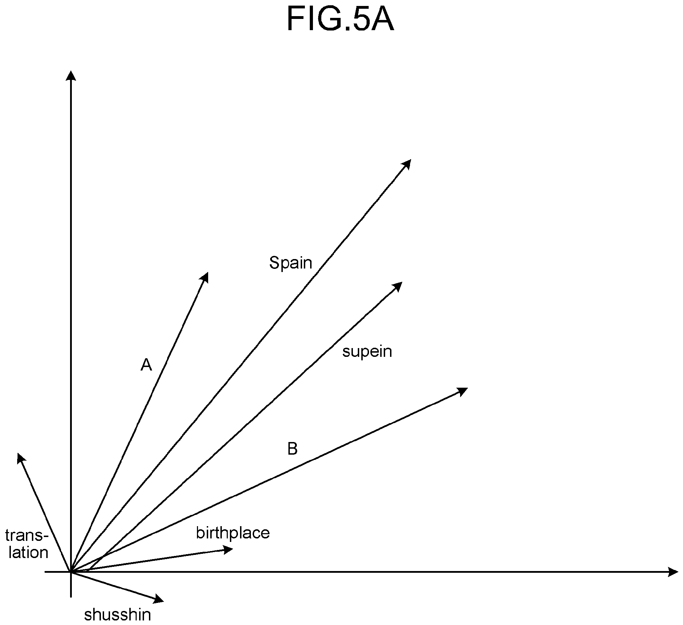

An example of a training process according to the embodiment will now be described by reference to FIG. 5A to FIG. 5H. FIG. 5A to 5H are diagrams illustrating the example of the training process according to the embodiment. A case where (subject, predicate, object) included in the RDF data 21 is (A, birthplace, Spain), (B, shusshin, supein), and (birthplace, translation, shusshin) will be described by reference to FIG. 5A to FIG. 5H. It is assumed that all of items in the RDF data 21 have already been initialized n-dimensionally. Herein, description will be made with respect to two-dimensional initialization for convenience.

As illustrated in FIG. 5A, the training unit 12 locates vectors that have been initialized by the initializing unit 11.

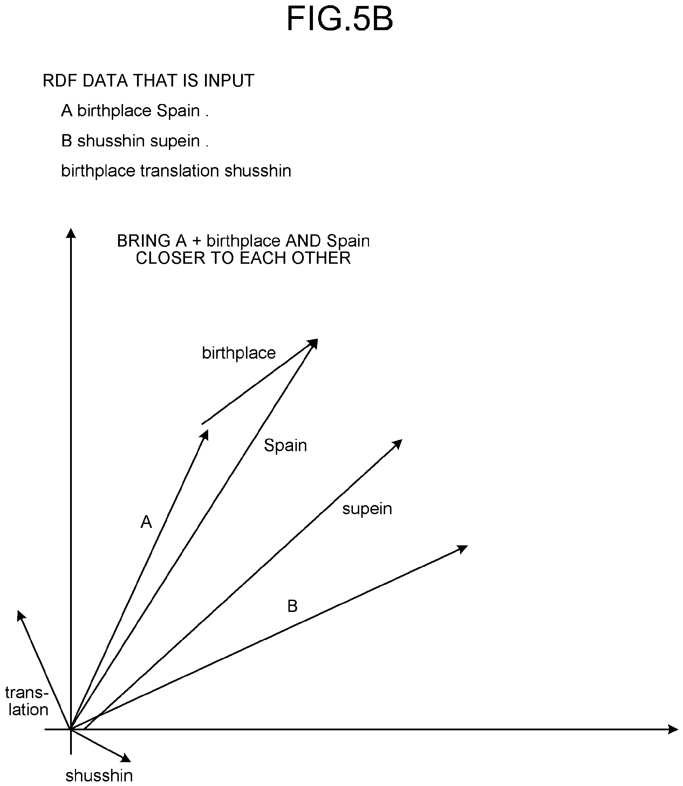

Next, the training unit 12 performs training so that a vector resulting from addition of the vector of the property indicating the predicate to the vector of the entity (synonymous with node) indicating the subject becomes closer to the vector of the entity (synonymous with node) indicating the object, for all of groups included in the RDF data 21. As illustrated in FIG. 5B, the training unit 12 brings the vector resulting from addition of the vector of the property, "birthplace", to the vector of the entity, "A", closer to the vector of the entity, "Spain".

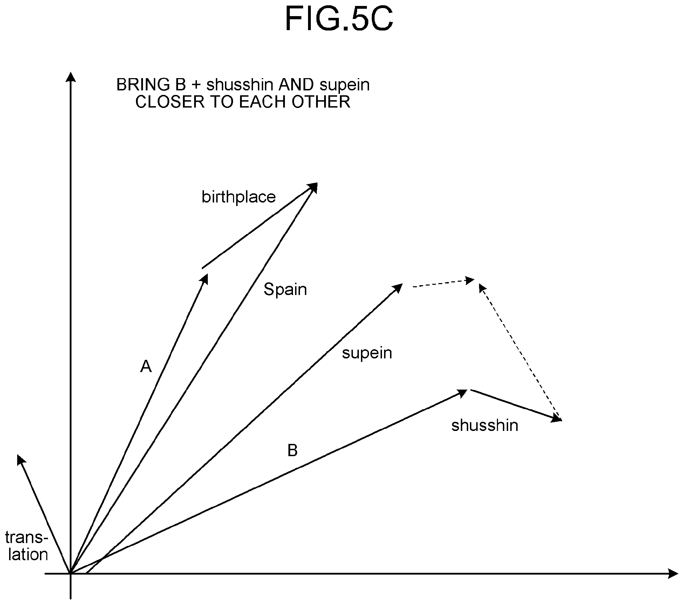

Next, as illustrated in FIG. 5C, the training unit 12 brings the vector resulting from addition of the vector of the property, "shusshin", to the vector of the entity, "B", closer to the vector of the entity, "supein".

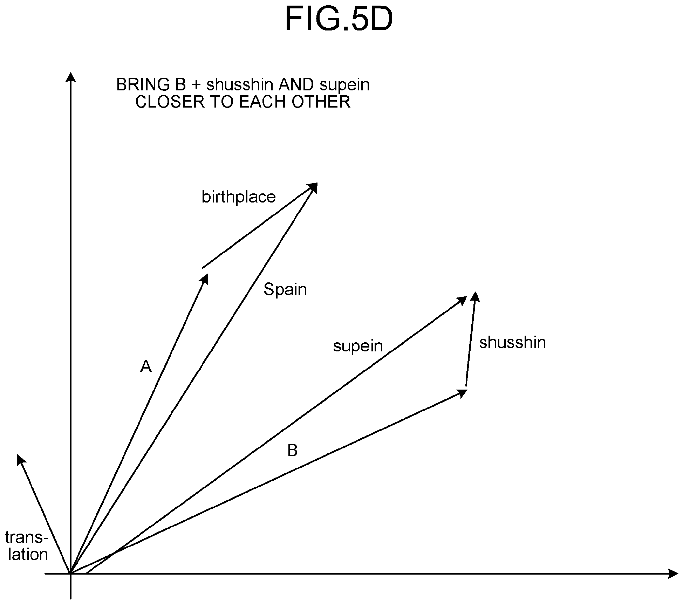

FIG. 5D illustrates a result of bringing the vector resulting from addition of the vector of the property, "shusshin", to the vector of the entity, "B", closer to the vector of the entity, "supein".

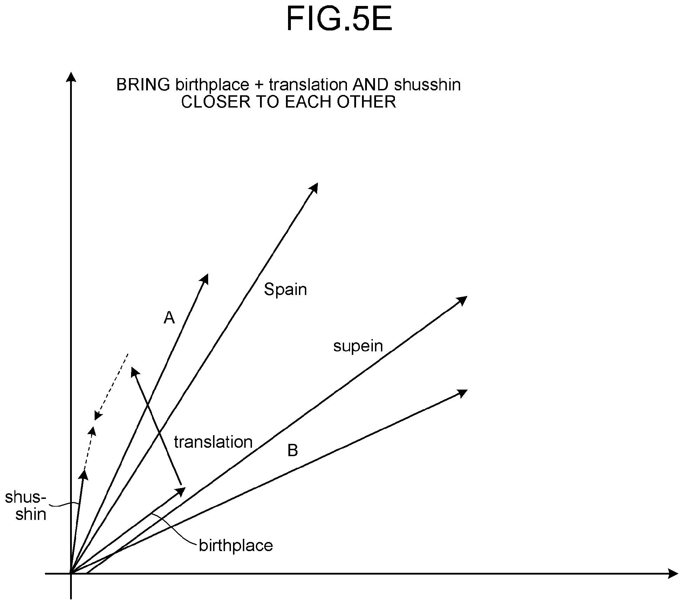

Next, as illustrated in FIG. 5E, the training unit 12 brings the vector resulting from addition of the vector of the property, "translation", to the vector of the entity, "birthplace", closer to the vector of the entity, "shusshin".

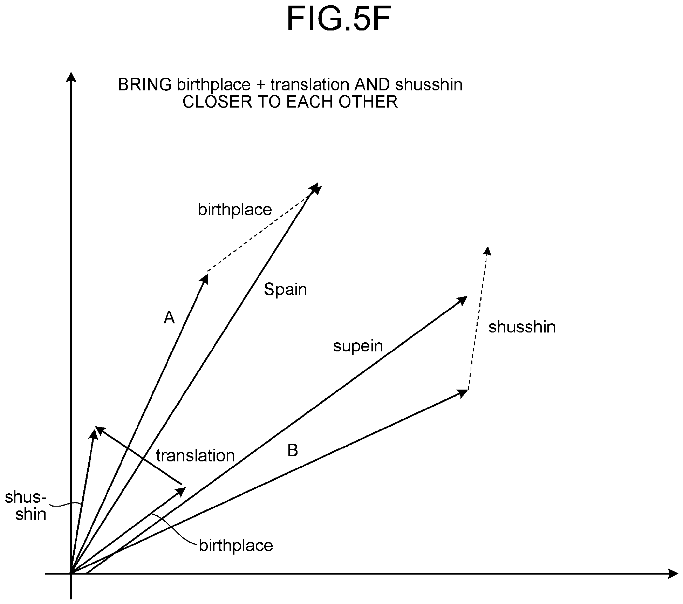

FIG. 5F illustrates a result of bringing the vector resulting from addition of the vector of the property, "translation", to the vector of the entity, "birthplace", closer to the vector of the entity, "shusshin". As a result, for example, the vector resulting from addition of the property, "shusshin", to the vector of the entity, "B", becomes distant from the vector of the entity, "supein".

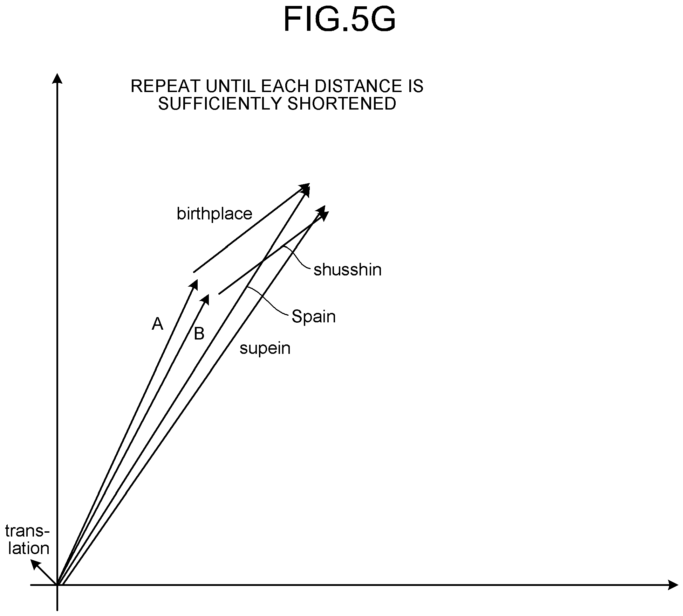

As illustrated in FIG. 5G, the training unit 12 thus repeats this process until each distance is sufficiently shortened. The number of times repeated is determined beforehand as one of hyperparameters.

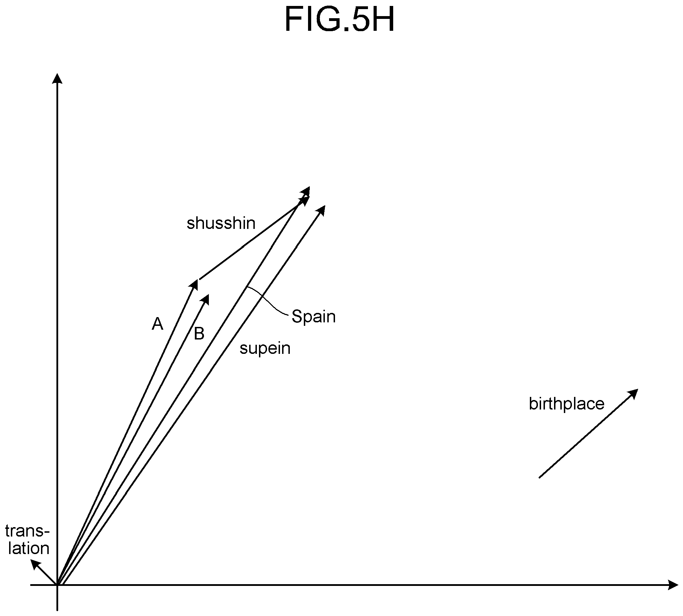

As a result, as illustrated in FIG. 5H, the training unit 12 generates, as a training result, vectors having sufficiently shortened distances from each other. The training unit 12 saves the training result in the training data 22. The training result is a set of trained vectors.

### Example of Predicting Process

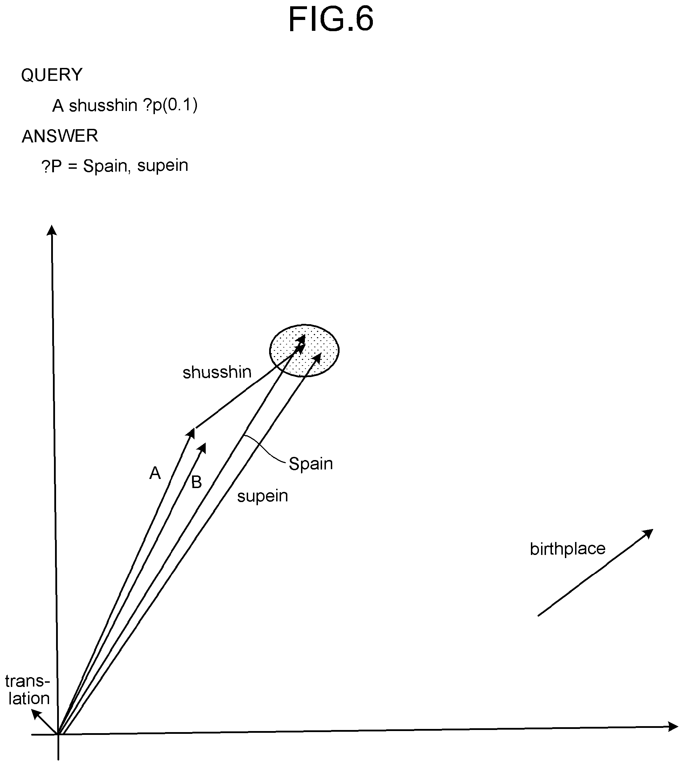

An example of a predicting process according to the embodiment will now be described by reference to FIG. 6. FIG. 6 is a diagram illustrating the example of the predicting process according to the embodiment. In FIG. 6, it is assumed that the training data 22 in which the set of trained vectors illustrated in FIG. 5H has been saved is to be used.

Description will be made with respect to a query, (A, shusshin, ?p(0.1)), on what "shusshin" of "A" is. In this query, "?" means a predictor variable indicating a prediction target. After the predictor variable, the value, "0.1" means a score indicating an allowable error of a vector. For example, the score refers to information indicating the inside of a circle illustrated in FIG. 6.

As illustrated in FIG. 6, the predicting unit 32 obtains vectors from the trained vectors corresponding to two character strings other than the prediction target for the input group. Herein, vectors respectively corresponding to "A" and "shusshin" are obtained.

Subsequently, the predicting unit 32 selects vectors one by one from the set of trained vectors. By using the vector selected and the vectors of the character strings other than the prediction target, the predicting unit 32 then retrieves a vector smaller than the score, the vector resulting from subtraction of a vector of an object from a vector resulting from addition of the vector of the predicate to the vector of the subject. Herein, for each vector selected, the predicting unit 32 determines whether a vector resulting from subtraction of the selected vector from the vector resulting from addition of the vector of "shusshin" indicating a predicate to the vector of "A" indicating a subject becomes a vector smaller than the score.

The predicting unit 32 predicts, as the prediction target, the character string corresponding to the vector that can be retrieved. Herein, "Spain" and "supein" are predicted as the prediction target.

The predicting unit 32 thereby enables improvement in prediction accuracy of searching. That is, "birthplace" that is an entity and "shusshin" that is an entity are in a "translation" relation. When "shusshin" that is a property is used as an entity, "shusshin", the vector representing the property, "shusshin", is represented by a vector that is the same as the vector representing the entity, "shusshin". When "birthplace" that is a property is used as an entity, "birthplace", the vector representing the property, "birthplace", is represented by a vector that is the same as the vector representing the entity, "birthplace". Therefore, an A-group, "A birthplace Spain" including the property, "birthplace", and a C-group, "birthplace translation shusshin" including the entity, "birthplace", will be located close to each other. In addition, a B-group, "B shusshin supein", including the property, "shusshin", and the C-group including the entity, "shusshin", are located close to each other. Therefore, the A-group including the property, "birthplace", and the B-group including the property, "shusshin", will be located close to each other, and in this case, "Spain" and "supein" having a translation relation will be predicted as "shusshin" of "A".

In other words, when a property is used as an entity, the vector representing the property is used in calculation for the entity. Therefore, the relation between the properties and the relation between the property and the entity are reflected in the vector of the property. The structure of the group of the property is used in the prediction and prediction accuracy of searching is increased.

### Flowchart of Training Process

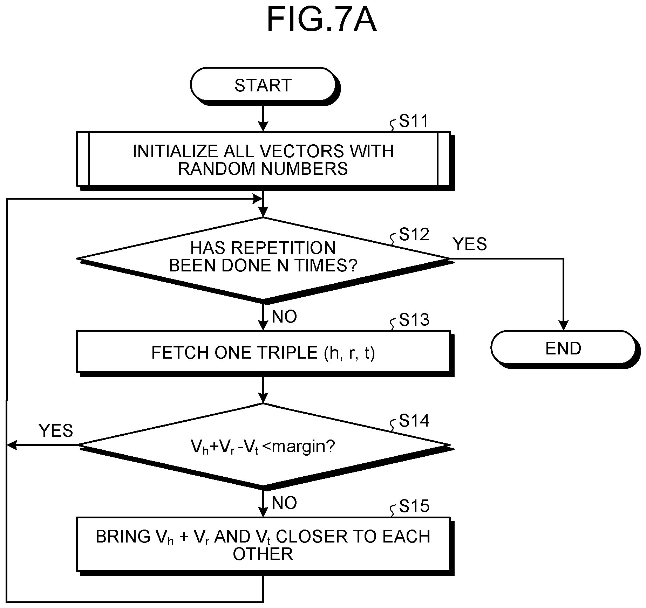

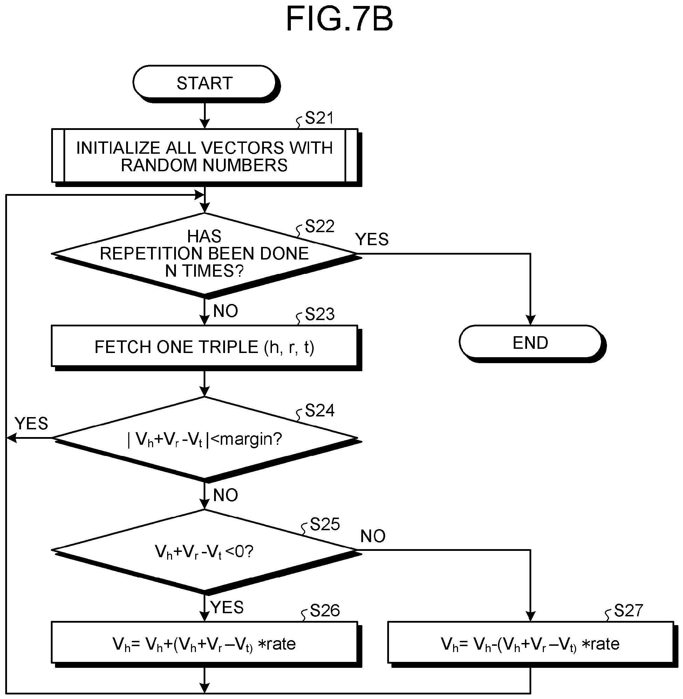

According to the embodiment, a flowchart of the training process will now be described by reference to FIG. 7A and FIG. 7B. In FIG. 7A and FIG. 7B, a hyperparameter indicating the maximum number of repetitions is denoted by N. A hyperparameter indicating a score is denoted by "margin". A hyperparameter indicating a correction rate at which a vector is corrected is denoted by "rate".

FIG. 7A is a diagram illustrating an example of a flowchart for the training process according to the embodiment. As illustrated in FIG. 7A, the initializing unit 11 initializes all of vectors corresponding to character strings included in the RDF data 21, with random numbers (Step S11). A flowchart for the initializing unit 11 will be described later.

The training unit 12 then determines whether or not repetition has been done N times, this N indicating the maximum number of repetitions (Step S12). In a case where the training unit 12 has determined that repetition has been done N times (Step S12; Yes), the training unit 12 ends the training process.

On the contrary, in a case where the training unit 12 has determined that repetition has not been done N times (Step S12; No), the training unit 12 fetches one triple (h, r, t) from the RDF data 21 (Step S13). Herein, h is an entity (a subject), r is a property (a predicate), and t is an entity (an object).

The training unit 12 then determines whether or not Vₕ + Vᵣ - Vₜ is smaller than margin (Step S14). That is, for the triple fetched and a triple that has been fetched previously, the training unit 12 determines whether or not a vector resulting from addition of the vector Vᵣ of the property r indicating a predicate to the vector Vₕ of the entity h indicating a subject becomes closer to a vector Vₜ of an entity t indicating an object.

In a case where the training unit 12 has determined that Vₕ + Vᵣ- Vₜ is not smaller than margin for any of the triples (Step S14; No), the training unit 12 brings Vₕ + Vᵣ closer to Vₜ (Step S15). The training unit 12 then proceeds to Step S12 to do the subsequent repetition.

On the contrary, in a case where the training unit 12 has determined that Vₕ + Vᵣ- Vₜ is equal to or smaller than margin for all of the triples (Step S14; Yes), the training unit 12 proceeds to Step S12 to do the subsequent repetition.

Considering a case where Vₕ + Vᵣ - Vₜ becomes negative, Vₕ + Vᵣ - Vₜ may be modified to |Vₕ + Vᵣ - Vₜ|. By reference to FIG. 7B, a case where Vₕ + Vᵣ - Vₜ becoming negative is considered will be described. FIG. 7B is a diagram illustrating another example of the flowchart for the training process according to the embodiment.

As illustrated in FIG. 7B, the initializing unit 11 initializes all of vectors corresponding to character strings included in the RDF data 21, with random numbers (Step S21). A flowchart for the initializing unit 11 will be described later.

The training unit 12 then determines whether or not repetition has been done N times, this N indicating the maximum number of repetitions (Step S22). In a case where the training unit 12 has determined that repetition has been done N times (Step S22; Yes), the training unit 12 ends the training process.

On the contrary, in a case where the training unit 12 has determined that repetition has not been done N times (Step S22; No), the training unit 12 fetches one triple (h, r, t) from the RDF data 21 (Step S23). Herein, h is an entity (a subject), r is a property (a predicate), and t is an entity (an object).

The training unit 12 then determines whether or not |Vₕ + Vᵣ - Vₜ| is smaller than margin (Step S24). That is, for the triple fetched and a triple that has been fetched previously, the training unit 12 determines whether or not a vector resulting from addition of the vector Vᵣ of the property r indicating a predicate to the vector Vₕ of the entity h indicating a subject becomes closer to a vector Vₜ of an entity t indicating an object.

In a case where the training unit 12 has determined, for any of the triples, that |Vₕ + Vᵣ - Vₜ| is not smaller than margin (Step S24; No), the training unit 12 determines whether or not Vₕ + Vᵣ - Vₜ is smaller than 0 (Step S25). In a case where the training unit 12 has determined that Vₕ + Vᵣ - Vₜ is smaller than 0 (Step S25; Yes), the training unit 12 determines a vector resulting from addition of (Vₕ + Vᵣ - Vₜ) × rate to Vₕ, as Vₕ (Step S26). That is, the training unit 12 corrects the vector to bring Vₕ + Vᵣ closer to Vₜ. The training unit 12 then proceeds to Step S22 to do the subsequent repetition.

On the contrary, in a case where the training unit 12 has determined that |Vₕ + Vᵣ - Vₜ| is equal to or larger than 0 (Step S25; No), the training unit 12 determines a vector resulting from subtraction of (Vₕ + Vᵣ - Vₜ) × rate from Vₕ, as Vₕ (Step S27). That is, the training unit 12 corrects the vector to bring Vₕ + Vᵣ closer to Vₜ. The training unit 12 then proceeds to Step S22 to do the subsequent repetition.

At Step S24, in a case where the training unit 12 has determined, for any of the triples, that |Vₕ + Vᵣ - Vₜ| is smaller than margin (Step S24; Yes), the training unit 12 proceeds to Step S22 to do the subsequent repetition.

### Flowchart of Initializing Process

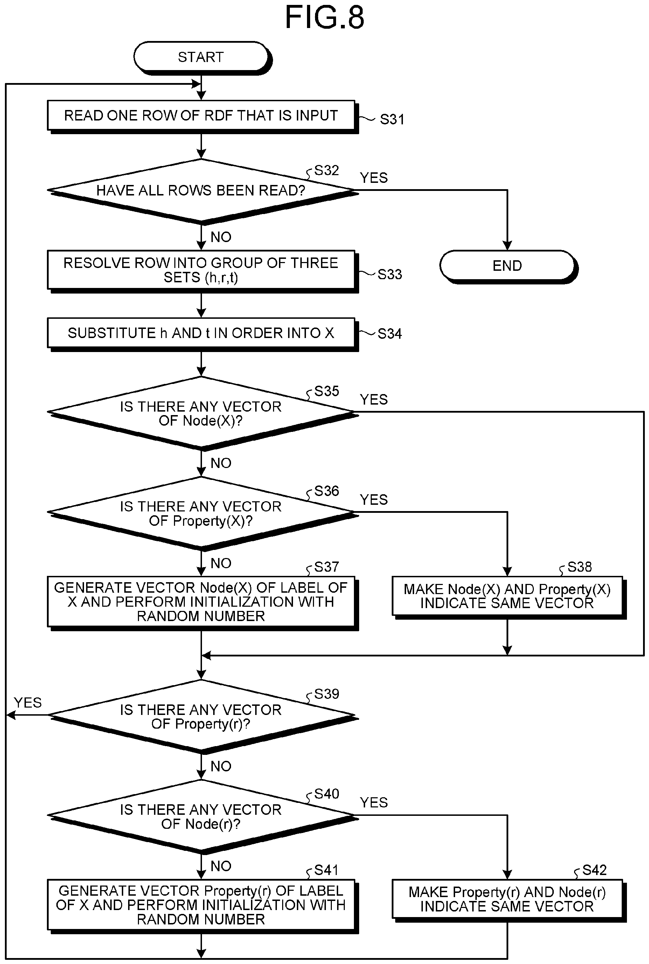

FIG. 8 is a diagram illustrating an example of a flowchart for the initializing process according to the embodiment. In the flowchart illustrated in FIG. 8, a triple, h, r, and t, represented by a row included in the RDF data 21 are respectively written as Node, Property, and Node, but these may be replaced with entity, property, and entity, or with subject, predicate, and object.

The initializing unit 11 reads one row of the RDF that is input from the RDF data 21 (Step S31). The initializing unit 11 determines whether or not all of the rows have been read (Step S32). In a case where the initializing unit 11 has determined that all of the rows have been read (Step S32; Yes), the initializing unit 11 ends the initializing process.

On the contrary, in a case where the initializing unit 11 has determined that not all of the rows have been read (Step S32; No), the initializing unit 11 resolves the read row into a group of three sets of data, (h, r, t) (Step S33).

The initializing unit 11 then substitutes h and t in order into X of the following step (Step S34). That is, the initializing unit 11 determines whether or not there is a vector of Node (X) for both h and t (Step S35). In a case where the initializing unit 11 has determined a vector of Node (X) for both h and t (Step S35; Yes), the initializing unit 11 proceeds to Step S39 to decide for the vector of r.

On the contrary, in a case where the initializing unit 11 has determined that there is no vector of Node (X) for both or any one of h and t (Step S35; No), the initializing unit 11 determines whether or not there is a vector of Property (X) (Step S36). In a case where the initializing unit 11 has determined that there is no vector of Property (X) (Step S36; No), the initializing unit 11 generates a vector Node (X) of a label of X and performs initialization with a random number (Step S37). The initializing unit 11 then proceeds to Step S39 to further determine the vector of r.

On the contrary, in a case where the initializing unit 11 has determined that there is a vector of Property (X) (Step S36; Yes), the initializing unit 11 makes Node (X) and Property (X) indicate the same vector (Step S38). The initializing unit 11 then proceeds to Step S39 to further determine the vector of r.

At Step S39, the initializing unit 11 determines whether or not there is a vector of Property (r) (Step S39). In a case where the initializing unit 11 has determined that there is a vector of Property (r) (Step S39; Yes), the initializing unit 11 proceeds to Step S31 to process the subsequent row.

On the contrary, in a case where the initializing unit 11 has determined that there is no vector of Property (r) (Step S39; No), the initializing unit 11 determines whether or not there is a vector of Node (r) (Step S40). In a case where the initializing unit 11 has determined that there is no vector of Node (r) (Step S40; No), the initializing unit 11 generates a vector Property (r) of a label of X and performs initialization with a random number (Step S41). The initializing unit 11 then proceeds to Step S31 to process the subsequent row.

On the contrary, in a case where the initializing unit 11 has determined that there is a vector of Node (r) (Step S40; Yes), the initializing unit 11 makes Property (r) and Node (r) indicate the same vector (Step S42). The initializing unit 11 then proceeds to Step S31 to process the subsequent row.

### Flowchart of Predicting Process

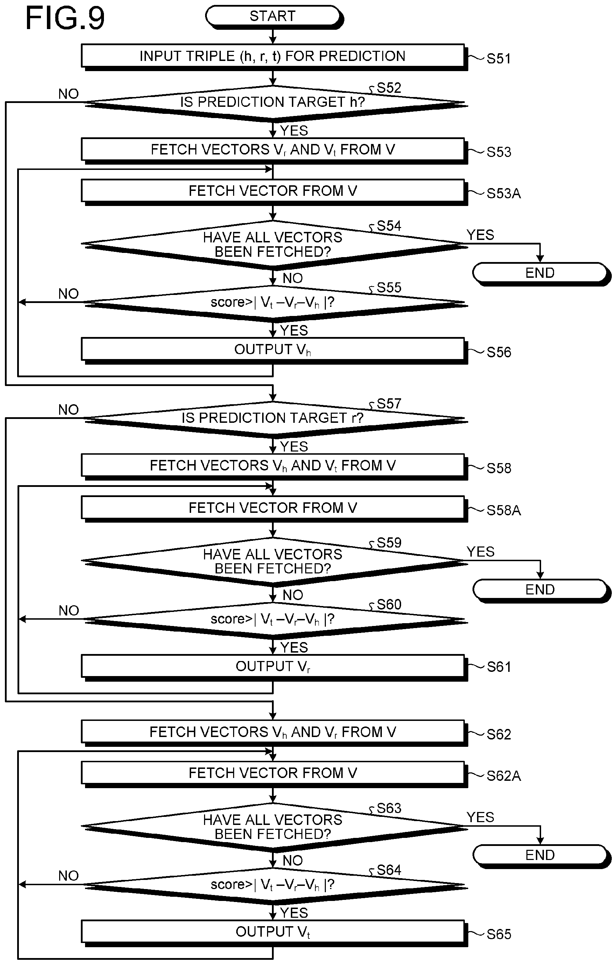

FIG. 9 is a diagram illustrating an example of a flowchart for the predicting process according to the embodiment. In FIG. 9, a hyperparameter indicating a score is denoted by score. Furthermore, the training data 22 trained by the training unit 12 has been generated. The training data 22 includes a set V of trained vectors.

As illustrated in FIG. 9, the input unit 31 inputs a triple (h, r, t), including a prediction target to be predicted (Step S51). The predicting unit 32 determines whether or not the prediction target is h (Step S52). In a case where the predicting unit 32 has determined that the prediction target is h (Step S52; Yes), the predicting unit 32 fetches vectors Vᵣ and Vₜ from the set V of trained vectors (Step S53). The predicting unit 32 fetches one vector from V (Step S53A). The predicting unit 32 then determines whether or not all of the vectors have been fetched from V (Step S54).

In a case where the predicting unit 32 determines that not all of the vectors have been fetched (Step S54; No), the predicting unit 32 determines whether or not |Vₜ - Vᵣ - Vₕ| is smaller than score, Vₕ being the vector fetched from V (Step S55). Herein, |Vₜ - Vᵣ - Vₕ| is synonymous with |Vₕ + Vᵣ - Vₜ|. When the predicting unit 32 determines that |Vt - Vr - Vh| is equal to or larger than the score (Step S55; No), the predicting unit 32 proceeds to Step S53A to fetch the subsequent vector.

On the contrary, in a case where the predicting unit 32 has determined that |Vₜ - Vᵣ - Vₕ| is smaller than score (Step S55; Yes), the output unit 33 outputs Vₕ as the prediction target (Step S56). The predicting unit 32 then proceeds to Step S53A to fetch the subsequent vector.

In a case where the predicting unit 32 determines at Step S54 that all of the vectors have been fetched (Step S54; Yes), the predicting unit 32 ends the predicting process.

In a case where the predicting unit 32 determines at Step S52 that the prediction target is not h (Step S52; No), the predicting unit 32 proceeds to Step S57.

At Step S57, the predicting unit 32 determines whether or not the prediction target is r (Step S57). In a case where the predicting unit 32 has determined that the prediction target is r (Step S57; Yes), the predicting unit 32 fetches vectors Vₕ and Vₜ from the set V of trained vectors (Step S58). The predicting unit 32 fetches one vector from V (Step S58A). The predicting unit 32 then determines whether or not all of the vectors have been fetched from V (Step S59).

In a case where the predicting unit 32 determines that not all of the vectors have been fetched (Step S59; No), the predicting unit 32 determines whether or not |Vₜ - Vᵣ - Vₕ| is smaller than score, Vᵣ being the vector fetched from V (Step S60). When the predicting unit 32 determines that |Vt - Vr - Vh| is equal to or larger than the score (Step S60; No), the predicting unit 32 proceeds to Step S58A to fetch the subsequent vector.

On the contrary, in a case where the predicting unit 32 has determined that |Vₜ - Vᵣ - Vₕ| is smaller than score (Step S60; Yes), the output unit 33 outputs Vᵣ as the prediction target (Step S61). The predicting unit 32 then proceeds to Step S58A to fetch the subsequent vector.

In a case where the predicting unit 32 determines at Step S59 that all of the vectors have been fetched (Step S59; Yes), the predicting unit 32 ends the predicting process.

In a case where the predicting unit 32 determines at Step S57 that the prediction target is not r (Step S57; No), the predicting unit 32 proceeds to Step S62.

At Step S62, the predicting unit 32 determines that the prediction target is t and fetches vectors Vₕ and Vᵣ from the set V of trained vectors (Step S62). The predicting unit 32 fetches one vector from V (Step S62A). The predicting unit 32 then determines whether or not all of the vectors have been fetched from V (Step S63).

In a case where the predicting unit 32 determines that not all of the vectors have been fetched (Step S63; No), the predicting unit 32 determines whether or not |Vₜ - Vᵣ - Vₕ| is smaller than score, Vₜ being the vector fetched from V (Step S64). When the predicting unit 32 determines that lVt - Vr - Vh| is equal to or larger than the score (Step S64; No), the predicting unit 32 proceeds to Step S62A to fetch the subsequent vector.

On the contrary, in a case where the predicting unit 32 has determined that |Vₜ - Vᵣ - Vₕ| is smaller than score (Step S64; Yes), the output unit 33 outputs Vₜ as the prediction target (Step S65). The predicting unit 32 then proceeds to Step S62A to fetch the subsequent vector.

In a case where the predicting unit 32 determines at Step S63 that all of the vectors have been fetched (Step S63; Yes), the predicting unit 32 ends the predicting process.

### Another Example of RDF Data

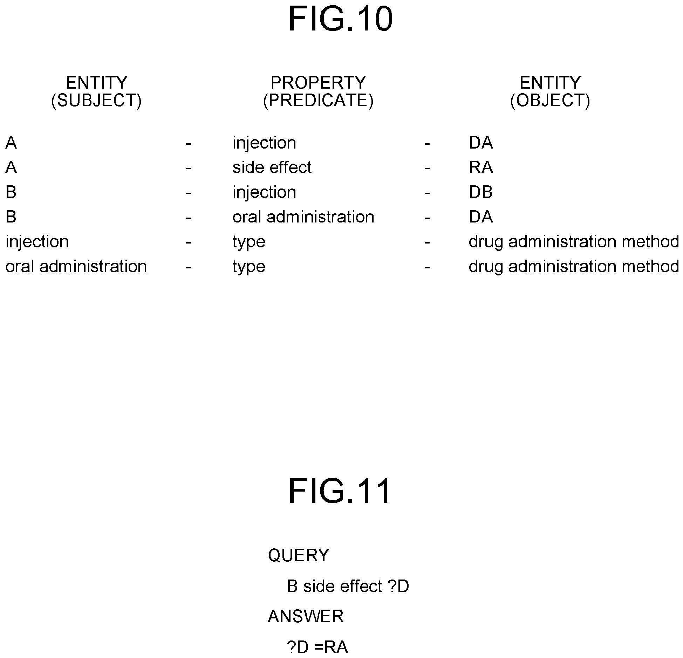

A predicting process in a case where RDF data according to an embodiment is applied to the pharmaceutical field will now be described by reference to FIG. 10 to FIG. 12. FIG. 10 is a diagram illustrating another example of the RDF data according to the embodiment. FIG. 10 illustrates RDF data 21 having three sets of data, an entity (a subject), a property (a predicate), and an entity (an object), as a group. Each group has the relation that "the predicate of the subject is the object".

For example, the RDF data 21 has (A, injection, DA) stored therein as (subject, predicate, object). This (A, injection, DA) has a relation that "'injection' of 'A' is 'DA'". (A, side effect. RA) has been stored as (subject, predicate, object). This (A, side effect. RA) has a relation that "'Side effect' of 'A' is 'RA'". (B, oral administration, DA) has been stored as (subject, predicate, object). This (B, oral administration, DA) has a relation that "'oral administration' of 'B' is 'DA'. (Injection, type, drug administration method) has been stored as (subject, predicate, object). This (injection, type, drug administration method) has a relation that "'type' of 'injection' is 'drug administration method'". (Drug administration, type, drug administration method) has been stored as (subject, predicate, object). This (drug administration, type, drug administration method) has a relation that "'type' of 'drug administration' is 'drug administration method'.

For all of these groups included in the RDF data 21, the training unit 12 performs training so that a vector resulting from addition of a vector of a property indicating a predicate to a vector of an entity indicating a subject becomes closer to a vector of an entity indicating an object. The training unit 12 then saves a result of this training in the training data 22. The training to be saved in the training data 22 includes a set of trained vectors for respective character strings included in subjects, predicates, and objects included in the RDF data 21.

By using this training data 22, the predicting unit 32 predicts an answer to a query. FIG. 11 is a diagram illustrating a predicting process using the other example of the RDF data.

The description will be made for a query (B, side effect, ?D) on what "side effect" of "B" is. In this query, "?" means a predictor variable indicating a prediction target.

As illustrated in FIG. 11, the predicting unit 32 obtains vectors corresponding to two character strings other than the prediction target of the input group, from the set of trained vectors. Herein, vectors respectively corresponding to "B" and "side effect" are obtained.

Subsequently, the predicting unit 32 selects vectors one by one from the set of trained vectors. By using the vector selected and the vectors of the character strings other than the prediction target, the predicting unit 32 then retrieves a vector smaller than a score, the vector resulting from subtraction of the vector of the object from the vector resulting from addition of the vector of the predicate to the vector of the subject. Herein, for each vector selected, the predicting unit 32 determines whether a vector resulting from subtraction of the selected vector from the vector resulting from addition of the vector of "side effect" indicating a predicate to the vector of "B" indicating a subject becomes a vector smaller than the score.

The predicting unit 32 then predicts, as the prediction target, the character string corresponding to the vector that has been able to be retrieved. Herein, "RA" is predicted as the prediction target.

The predicting unit 32 thereby enables improvement in prediction accuracy of searching. That is, "oral administration" that is an entity and "drug administration method" that is an entity have a relation of "type" that is a property. In addition, "injection" that is an entity and "drug administration method" that is an entity has a relation of "type" that is a property. Therefore, "injection" that is an entity and "oral administration" that is an entity become approximate vectors because their predicate, "type", and their object, "drug administration method" are common to them. "Injection" that is an entity and "injection" that is the same vector represents a property, "oral administration" that is an entity and "oral administration" that is a property are represented by the same vector, and this "injection" that is a property and this "drug administration" that is a property thus become approximate vectors. Therefore, "B oral administration DA" including "oral administration" that is a property and "A injection DA" including "injection" that is a property will be located close to each other. As a result, in this case, because "side effect" of "A" is "RA", "RA" is predicted as "side effect" of "B".

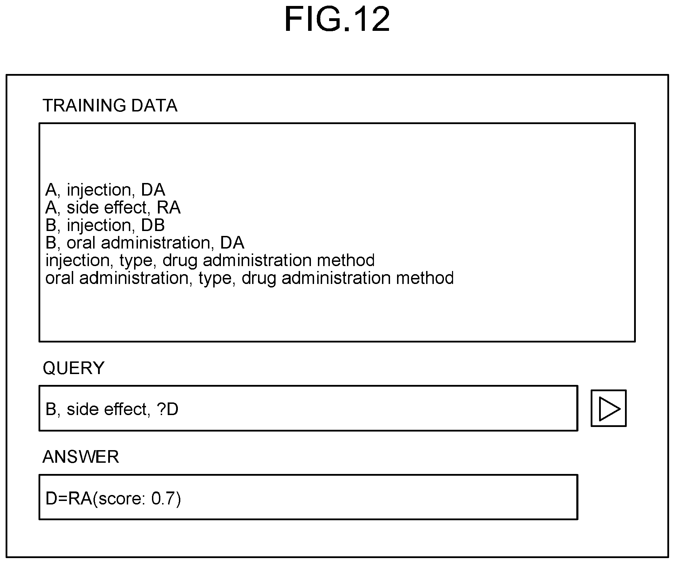

The output unit 33 then outputs the prediction target predicted by the predicting unit 32, to the user terminal 5. FIG. 12 is a diagram illustrating an example of an output screen according to the embodiment.

The output screen has, displayed thereon, a field for output of training data, a field for input of a query, and a field for output of an answer. The field for output of training data has displayed each group in the RDF data 21.

For example, when a user inputs, as a query, "B, side effect, ?D" in the field for input of a query, "D = RA (score: 0.7)" is output in the field for output of an answer. Herein, "0.7" means a score indicating an allowable error for the vector.

### Example of Training Model

The training unit 12 has been described above to perform training so that a vector resulting from addition of a vector of a property indicating a predicate to a vector of an entity indicating a subject becomes closer to a vector of an entity indicating an object, for all of the groups included in the RDF data 21. In this training, in a case where the character string of the property indicating the predicate of a group has been associated with the character string of the entity indicating the subject or object of another group, the training unit 12 calculates, as the same vector, the property and the entity of these character strings associated with each other, when training vectors. The training unit 12 may execute such a training process by using a neural network. That is, by using a neural network, the training unit 12 may perform training so that a vector resulting from addition of the vector of a property indicating a predicate to the vector of the entity indicating a subject becomes closer to a vector of an entity indicating an object, for all of the groups included in the RDF data 21. In this training, in a case where the character string of the property indicating the predicate of a group has been associated with the character string of the entity indicating the subject or object of another group, the training unit 12 calculates, as the same vector, the property and the entity of these character strings associated with each other, when training vectors.

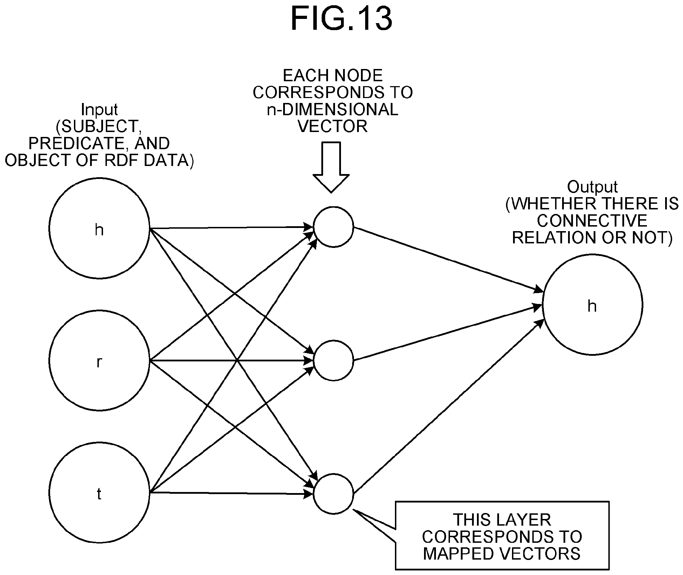

According to the embodiment, FIG. 13 is a diagram illustrating how training is performed using a neural network in the training process. FIG. 13 illustrates a training model of the neural network. The training model is a model where a subject, a predicate, and an object of a group included in the RDF data 21 are input and whether or not there is a connective relation between the input subject, predicate, and object is output. Respective nodes of a layer in the training model correspond to n-dimensional vectors.

For example, it is assumed that groups (subject, predicate, object) included in the RDF data 21 are (A, birthplace, Spain) and (birthplace, translation, shusshin). The training unit 12 inputs (A, birthplace, Spain) into the training model and trains the training model to generate a vector to have a connective relation. That is, the training unit 12 trains the training model so that a vector resulting from addition of the vector of "birthplace" indicating a predicate to the vector of "A" indicating a subject becomes closer to the vector of "Spain" indicating an object. Furthermore, the training unit 12 inputs (birthplace, translation, shusshin) into the training model and trains the training model to generate a vector so that there will be a connective relation. That is, the training unit 12 trains the training model such that a vector resulting from addition of the vector of "translation" indicating a predicate to the vector of "birthplace" indicating a subject becomes closer to the vector of "shusshin" indicating an object. In training the training model, because the character string for "birthplace" of the property and the character string for "birthplace" of the entity of the other group are the same, the training unit 12 calculates the property and entity of the character string, "birthplace", as the same vector.

By using the trained training model that has been trained as described above, the predicting unit 32 may predict a prediction target for the input group. That is, the predicting unit 32 obtains two character strings other than the prediction target, from the input group. The predicting unit 32 then selects character strings one by one from the set of character strings in the RDF data 21. By using the two-character strings obtained and the selected character string, the predicting unit 32 inputs these character strings as a character string of a subject, a character string of a predicate, and a character string of an object, so that whether or not there is a connective relation is output. The predicting unit 32 may predict, as the prediction target, the selected character string that is output as having a connective relation.

### Effects of Embodiments

According to the above described embodiments, the training apparatus 1 obtains RDF data including subjects, predicates, and objects. The training apparatus 1 inputs a subject, a predicate, and an object of a first record in the RDF data obtained. The training apparatus 1 determines a predicate of a second record that has been input previously. The predicate has the same character string as the subject or object of the first record. The training apparatus 1 generates training data formed of RDF data having the subject or object of the first record and the determined predicate of the second record that have been associated with each other. The training apparatus 1 performs training for the generated training data so that a vector resulting from addition of the vector of the predicate to the vector of the subject associated with the RDF data becomes closer to the vector of the object associated with the RDF data. This configuration enables improvement in prediction accuracy of searching through the RDF data by using the training data in the training of TransE, the training data being formed of the RDF data having the subject or object of the first record in the RDF data and the determined predicate of the second record that have been associated with each other. For example, in a case where there are three records, (A, birthplace, Spain), (B, shusshin, supein), and (birthplace, translation, shusshin), in the RDF data, "birthplace" of the predicate and "birthplace" of the subject are associated with each other to have the same vector, "shusshin" of the predicate and "shusshin" of the object are associated with each other to have the same vector, and these three records are thereby able to be located close to one another and "shusshin" of "A" is thus able to be predicted to be "Spain" and "supein".

Furthermore, according to the above described embodiments, the training apparatus 1 generates training data formed of the RDF data having the subject or object of the first record and the determined predicate of the second record that have been associated each other so that they have the same vector, the subject or object and the determined predicate having the same character string. According to this configuration, the training apparatus 1 is able to use the training data in the training of TransE, the training data being formed of the RDF data having the subject or object of the first record in the RDF data and the determined predicate of the second record that have been associated with each other so that the subject or object and the determined predicate have the same vector. Therefore, the training apparatus 1 improves prediction accuracy of searching through RDF data.

Furthermore, according to the embodiment, the training apparatus 1 inputs a subject, a predicate, and an object that are associated with RDF data, performs training for the generated training data so that a vector resulting from addition of the vector of the predicate to the vector of the subject associated with the RDF data becomes closer to the vector of the object associated with the RDF data, and generates a training model that outputs whether or not there is a nearing relation. According to this configuration, the training apparatus 1 is able to predict a subject, a predicate, and an object having a nearing relation with each other by using a training model.

Furthermore, according to the embodiment, the predicting apparatus 3 predicts a prediction target for which a training result is unknown, based on a result of training of RDF data, in response to input of a subject, a predicate, and an object, any one of which is the prediction target. According to this configuration, by using a result of training of RDF data, the predicting apparatus 3 enables improvement in prediction accuracy of searching through RDF data.

Furthermore, according to the embodiment, the predicting apparatus 3 selects vectors one by one from trained vectors included in a result of training of RDF data. By using the selected vector and vectors of predetermined parts other than a prediction target, the predicting apparatus 3 determines whether or not a vector is smaller than a predetermined score, the vector resulting from subtraction of the vector corresponding to the object from a vector resulting from addition of the vector corresponding to the predicate to the vector corresponding to the subject. The predicting apparatus 3 has, as the prediction target, the character string corresponding to the selected vector that has been determined to be smaller. According to this configuration, by using trained vectors included in a result of training of RDF data, the predicting apparatus 3 enables improvement in prediction accuracy of searching through the RDF data.

Furthermore, according to the embodiment, by using a trained model included in a result of training of RDF data, the predicting apparatus 3 predicts whether or not there is a nearing relation, from: a subject, a predicate, or an object other than a prediction target, among the subject, predicate, and object that have been input; and the character strings of the plural subjects, predicates, or objects included in the RDF data, and determines, as the prediction target, the character string predicted as having a nearing relation. According to this configuration, by using a trained model included in a result of training of RDF data, the predicting apparatus 3 enables improvement in prediction accuracy of searching through the RDF data.

Furthermore, according to the embodiment, the predicting apparatus 3 predicts a side effect, for which a training result is unknown, by having a medicinal drug name as an input, on the basis of a result of training of RDF data related to medical treatment, the RDF data being formed of: subjects related to adverse drug-reaction report cases; predicates related to patients, diseases, or medicinal drugs; and objects related to patient attributes, disease names, medicinal drug names, or known side effects. According to this configuration, by using a result of training of RDF data, the predicting apparatus 3 enables improvement in prediction accuracy of searching through RDF data related to medical treatment, for a side effect, from a medicinal drug name.

### Others

Components of the training apparatus 1 and the predicting apparatus 3 that have been illustrated are not necessarily configured physically as illustrated in the drawings. That is, specific modes of separation and integration of the training apparatus 1 and predicting apparatus 3 are not limited to those illustrated in the drawings, and all or a part thereof may be configured by functionally or physically separating or integrating the apparatuses in any units depending on various loads and use situations. For example, the initializing unit 11 and the training unit 12 may be integrated into a single unit. Furthermore, the storage unit 20 may be connected via a network as an external device of the training apparatus 1. The storage unit 40 may be connected via a network as an external device of the predicting apparatus 3.

Furthermore, a configuration in which the training apparatus 1 performs the training process and the predicting apparatus 3 performs the predicting process are separated has been described with respect to the embodiments. However, an information processing apparatus may be configured to include the training process and the predicting process.

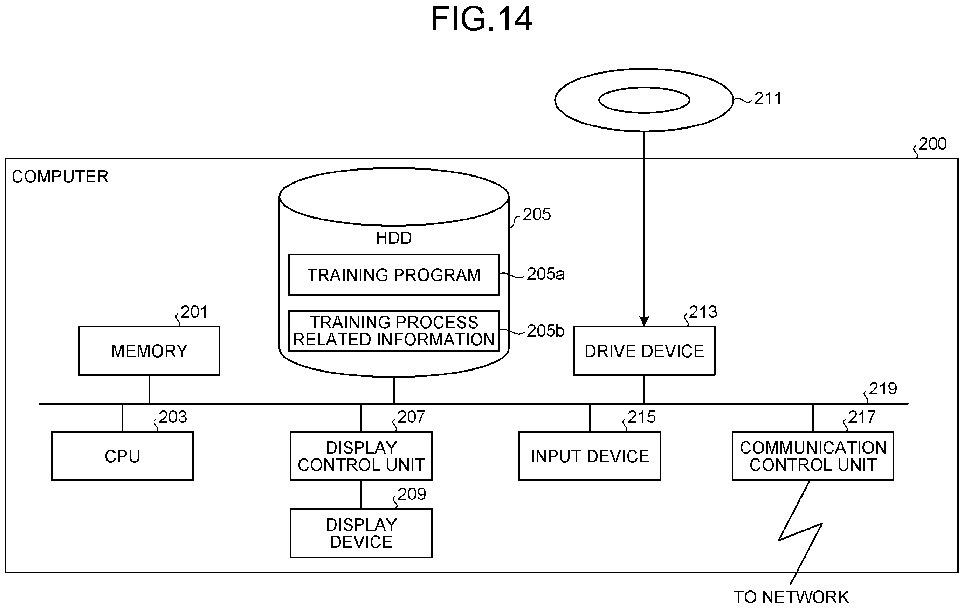

Furthermore, various processes described with respect to the embodiments may be implemented by a computer, such as a personal computer or a work station, executing a program that has been prepared beforehand. An example of a computer that executes a training program that implements functions similar to those of the training apparatus 1 illustrated in FIG. 1 and a prediction processing program that implements functions similar to those of the predicting apparatus 3 will thus be described hereinafter. A training program that implements functions similar to those of the training apparatus 1 will be described herein as an example. FIG. 14 is a diagram illustrating an example of a computer that executes the training program.

As illustrated in FIG. 14, a computer 200 has a CPU 203 that executes various types of arithmetic processing, an input device 215 that receives input of data from a user, and a display control unit 207 that controls a display device 209. Furthermore, the computer 200 has a drive device 213 that reads a program, for example, from a storage medium, and a communication control unit 217 that transfers data to and from another computer via a network. In addition, the computer 200 has a memory 201 that temporarily stores therein various types of information, and a hard disk drive (HDD) 205. The memory 201, the CPU 203, the HDD 205, the display control unit 207, the drive device 213, the input device 215, and the communication control unit 217 are connected to one another by a bus 219.

For example, the drive device 213 is a device for a removable disk 210. The HDD 205 stores a training program 205a and training process related to information 205b.

The CPU 203 reads the training program 205a, loads the read training program 205a into the memory 201, and executes the training program 205a as a process. This process corresponds to the respective functional units of the training apparatus 1. The training process related information 205b corresponds to the RDF data 21 and the training data 22. For example, the removable disk 210 stores therein information, such as the training program 205a.

The training program 205a has not necessarily been stored in the HDD 205 from the start. For example, the program is stored in a "portable physical medium", such as a flexible disk (FD), a compact disk read only memory (CD-ROM), a digital versatile disk (DVD), a magneto-optical disk, or an integrated circuit (IC) card, which is inserted into the computer 200. The computer 200 may then read and execute the training program 205a therefrom.

### [Explanation of Reference]

- 1: TRAINING APPARATUS

- 10: CONTROL UNIT
- 11: INITIALIZING UNIT
- 12: TRAINING UNIT
- 20: STORAGE UNIT
- 21: RDF DATA
- 22: TRAINING DATA
- 3: PREDICTING APPARATUS
- 30: CONTROL UNIT
- 31: INPUT UNIT
- 32: PREDICTING UNIT
- 33: OUTPUT UNIT
- 40: STORAGE UNIT
- 41: TRAINING DATA
- 5: USER TERMINAL
- 9: TRAINING SYSTEM

## Claims

1. A training method for causing a computer to execute a process comprising:
obtaining resource description framework (RDF) data including subjects, predicates, and objects;
inputting a subject, a predicate, and an object of a first record in the RDF data obtained;
determining a predicate of a second record previously input, the predicate having the same character string as the subject or the object of the first record;
generating training data including RDF data having the subject or the object of the first record and the determined predicate of the second record that have been associated with each other; and
performing training for the generated training data so that a vector resulting from the addition of a vector of the predicate to a vector of the subject associated with the RDF data becomes closer to a vector of the object associated with the RDF data.

2. The training method according to claim 1, wherein the generating includes generating the training data from the RDF data having the subject or the object of the first record and the determined predicate of the second record that have been associated with each other so that the vector of the subject or the object of the first record and the vector of the determined predicate of the second record become the same vector, the subject or the object of the first record and the determined predicate of the second record having the same character string.

3. The training method according to claim 1, wherein the training includes: inputting a subject, a predicate, and an object that have been associated with the RDF data; performing training, for the training data generated, so that the vector resulting from addition of the vector of the predicate to the vector of the subject associated with the RDF data becomes closer to the vector of the object associated with the RDF data; and generating a training model that outputs whether or not there is a nearing relation.

4. The training method according to claim 1, further comprising predicting, in response to input of input data for a subject, a predicate, and an object, the input data having one of a subject, a predicate, or an object as a prediction target, the prediction target for which a training result is unknown on the basis of a result of training of the RDF data.

5. The training method according to claim 4, wherein the predicting includes:
selecting a vector one by one from trained vectors included in the result of the training of the RDF data;
determining, by using the selected vector and vectors of predetermined parts other than the prediction target, whether or not a vector is smaller than a predetermined score, the vector resulting from subtraction of a vector corresponding to an object from a vector resulting from addition of a vector corresponding to a predicate to a vector corresponding to a subject; and
predicting, as the prediction target, a character string corresponding to the selected vector that has been determined to be smaller.

6. The training method according to claim 4, wherein the predicting includes: determining whether or not there is a nearing relation, from the subject, the predicate, or the object other than the prediction target, of the input subject, predicate, and object, by using a training model included in the result of the training of the RDF data; and identifying, as the prediction target, a character string that is output as having the nearing relation.

7. The training method according to any one of claim 4 to claim 6, wherein
the predicting includes predicting, based on the result of the training of the RDF data, a side effect for which a training result is unknown, by inputting a medicinal drug name, the RDF data is RDF data that is related to medical treatment and that includes: subjects related to adverse drug-reaction report cases; predicates related to patients, diseases, and/or medicinal drugs; and objects related to patient attributes, disease names, medicinal drug names, and/or known side effects.

8. A training apparatus, comprising:
an obtaining unit that obtains resource description framework (RDF) data including subjects, predicates, and objects;
a input unit that inputs a subject, a predicate, and an object of a first record of the RDF data obtained;
a determining unit that determines a predicate of a second record that has been input previously, the predicate having the same character string as the subject or the object of the first record;
a generating unit that generates training data including RDF data having the subject or the object of the first record and the determined predicate of the second record that have been associated with each other; and
a training unit that performs training for the generated training data so that a vector resulting from addition of a vector of the predicate to a vector of the subject associated with the RDF data becomes closer to a vector of the object associated with the RDF data.

9. A training program that causes a computer to execute a process comprising:
obtaining resource description framework (RDF) data including subjects, predicates, and objects;
inputting a subject, a predicate, and an object of a first record of the RDF data obtained;
determining a predicate of a second record that has been input previously, the predicate having the same character string as the subject or the object of the first record;
generating teacher data including RDF data having the subject or the object of the first record and the determined predicate of the second record that have been associated with each other; and
performing training for the generated teacher data so that a vector resulting from addition of a vector of the predicate to a vector of the subject associated with the RDF data becomes closer to a vector of the object associated with the RDF data.

10. A predicting method for causing a computer to execute a process comprising:
inputting of input data for a subject, a predicate, and an object, the input data being from resource description framework (RDF) data including subjects, predicates, and objects, the input data having, as a prediction target, one of a subject, a predicate, or an object;and
predicting a character string of the prediction target on the basis of a result of training of the RDF data, the training being for teacher data including the RDF data having a subject or an object of a first record in the RDF data and a predicate of a second record in the RDF data, the predicate having the same character string as the subject or the object of the first record, the subject or the object of the first record and the predicate of the second record having been associated with each other, the training being performed so that a vector resulting from addition of a vector of the predicate to a vector of the subject associated with the RDF data becomes closer to a vector of the object associated with the RDF data.

11. A predicting apparatus, comprising:
an input unit that inputs input data for a subject, a predicate, and an object, the input data being from resource description framework (RDF) data including subjects, predicates, and objects, the input data having, as a prediction target, one of a subject, a predicate, or an object; and
a predicting unit that predicts a character string of the prediction target on the basis of a result of training of the RDF data, the training being for teacher data including the RDF data having a subject or an object of a first record in the RDF data and a predicate of a second record in the RDF data, the predicate having the same character string as the subject or the object of the first record, the subject or the object of the first record and the predicate of the second record having been associated with each other, the training being performed so that a vector resulting from addition of a vector of the predicate to a vector of the subject associated with the RDF data becomes closer to a vector of the object associated with the RDF data.

12. A predicting program that causes a computer to execute a process comprising:
inputting of input data for a subject, a predicate, and an object, the input data being from resource description framework (RDF) data including subjects, predicates, and objects, the input data having, as a prediction target, one of a subject, a predicate, or an object;and
predicting a character string of the prediction target on the basis of a result of training of the RDF data, the training being for training data including the RDF data having a subject or an object of a first record in the RDF data and a predicate of a second record in the RDF data, the predicate having the same character strings as the subject or the object of the first record, the subject or the object of the first record and the predicate of the second record having been associated with each other, the training being performed so that a vector resulting from addition of a vector of the predicate to a vector of the subject associated with the RDF data becomes closer to a vector of the object associated with the RDF data.
